# EUROPÄISCHE PATENTSCHRIFT

(11) **EP 1 208 374 B1**
(45) Veröffentlichungstag und Bekanntmachung des Hinweises auf die Patenterteilung: **21.07.2004**
(21) Anmeldenummer: 01933666.8
(22) Anmeldetag: 07.03.2001
(51) Int. Cl.: G01N 33/18, G01N 33/24, G01N 33/00

(54) **VERFAHREN UND VORRICHTUNG ZUR SCHNELLEN UND KONTINUIERLICHEN ERFASSUNG VON ÄNDERUNGEN DER KONZENTRATION VON IN WASSER GELÖSTEM RADON-GAS**
METHOD AND DEVICE FOR QUICKLY AND CONTINUALLY DETECTING CHANGES IN THE CONCENTRATION OF RADON GAS THAT IS DISSOLVED IN WATER
PROCEDE ET DISPOSITIF POUR DETECTER RAPIDEMENT ET EN CONTINU DES MODIFICATIONS DE LA CONCENTRATION DU GAZ RADON DISSOUS DANS DE L'EAU

(30) Priorität: 08.03.2000 DE 10010558
(43) Veröffentlichungstag der Anmeldung: 29.05.2002
(73) Patentinhaber: UFZ Umweltforschungszentrum Leipzig-Halle GmbH, 04318 Leizig (DE)
(72) Erfinder: FREYER, Klaus, 04454 Holzhausen (DE); TREUTLER, Hanns-Christian, 04683 Naunhof (DE); JUST, Günther, 04463 Grosspösna (DE)
(74) Vertreter: Hengelhaupt, Jürgen, Dipl.-Ing.
(86) Internationale Anmeldenummer: PCT/EP2001/002566
(87) Internationale Veröffentlichungsnummer: WO 2001/066227

(56) Entgegenhaltungen:
- DE-A- 4 310 096
- US-A- 4 868 546
- US-A- 5 194 158
- LABED V.; RANNOU A.; TYMEN G.: 'Study of 222Rn Permeation through Polymer Membranes: Application to Continuous Measurement of 222Rn in Water' HEALTH PHYSICS Bd. 63, Nr. 2, August 1992, USA, Seiten 172 - 178
- SURBECK H.: 'A Radon-in-Water Monitor Based on Fast Gas Transfer Membranes' INT. CONF. TECHNOLOGICALLY ENHANCED NATURAL RADIOACTIVITY CAUSED BY NON-URANIUM MINING 16 Oktober 1996, Seiten 177 - 191

## Beschreibung

Die Erfindung betrifft ein Verfahren zur kontinuierlichen und insbesondere schnellen Erfassung von Änderungen der Konzentration von in Wasser gelöstem Radon-Gas durch Überführung in ein Meßgas (Rn-222), welches für vielfältige Überwachungs-, Kontroll- und Regelaufgaben herangezogen werden kann.

Sie betrifft auch eine spezielle Vorrichtung zur schnellen und kontinuierlichen Überführung von im Wasser gelöstem Radon-Gas (Rn-222) in ein Meßgas und dessen Weiterleitung in extra hierfür angefertigte oder handelsübliche Detektionssysteme für Radon-Gas (Rn-222).

Das natürliche radioaktive Edelgas Radon (Rn-222) entsteht überall und ständig neu, wenn das in der Zerfallsreihe von Uran-238 gebildete Radium-226 zerfällt. Da alle geologischen Materialien mehr oder weniger Uran enthalten, ist auch Radon allgegenwärtig und findet sich in unterschiedlichen Konzentrationen auch im Wasser. Für die meßtechnische Erfassung der Konzentration von Radon im Wasser existieren zahlreiche vom Wirkprinzip her unterschiedliche Methoden, welche im Feld, aber auch im Labor eingesetzt werden können.

Die kontinuierliche Erfassung auftretender Konzentrationsänderungen von Radon im Wasser ist für zahlreiche mögliche Anwendungen von großem Interesse. Die mit verschiedenen Verfahren bisher realisierten zeitlichen Auflösungen erweisen sich allerdings in vielen Fällen als nicht ausreichend bzw. als ungeeignet, so daß die gewünschten Informationen nicht oder nur unzureichend zur Verfügung gestellt werden konnten.

Als Beispiele für den möglichen Einsatz sollen hier stellvertretend die Optimierung der Probennahme von Grundwassermeßstellen, die Überwachung der Radonführung im Quellwasser als einer von mehreren Einträgen zur Erdbebenforschung und weiterhin die Kontrolle balneologischer Anwendungen von Radon im Wasser (Qualitätssicherung) genannt werden.

Bekannt ist die kontinuierliche Messung der Konzentration von Radon (Rn-222) in Wasser durch Überführung des Radons aus dem Wasser durch eine wasserdichte, gasdurchlässige Membran, z.B. in Form eines Schlauches, in einen Gaskreislauf (z.B. Luft), der durch ein Radonmeßgerät geleitet wird, in dem die Radonkonzentration in dem Gaskreislauf durch Messung der Aktivität des Radons und seiner Folgeprodukte bestimmt wird. (H. Surbeck, A Radon-in Water Monitor Based on Fast Gas Transfer Membranes, Int. Conf. Technologically Enhanced Natural Ratioactivity (TENR) Caused by Non-uranium Mining, October 16-19, 1996, Szczyrk, Poland). In dem geschlossenen Gaskreislauf baut sich, zeitlich entsprechend den Halbwertszeiten der Folgeprodukte verzögert, eine der Radonkonzentration im Wasser proportionale Aktivitätskonzentration auf.

Der Mangel dieser Vorrichtung ist, daß infolge dieser zeitlichen Verzögerung des Aktivitätsaufbaus eine kontinuierliche Bestimmung der Radonkonzentration im Wasser, insbesondere eine Konzentrationsänderung, nur mit einer zeitlichen Auflösung größer 15 min möglich ist.

Weiterhin beschreibt die WO 97/43637 ein Verfahren und Vorrichtungen zur Charakterisierung von Grundwassermeßstellen durch Unterscheidung von Grundwasser und Standwasser und dient beispielsweise zur Bestimmung der für repräsentative Beschaffenheitsuntersuchungen optimalen Abpumpzeiten von Grundwassermeßstellen sowie der Erkennung und Lokalisierung von Defekten an Grundwassermeßstellen. Die Erfindung basiert auf der Messung der Radonaktivitätskonzentration bzw. der Gesamtaktivitätskonzentration der Grundwasserproben.

Die Messung der Konzentrationen gestattet die Bestimmung des Verhältnisses von Grundwasser zu Standwasser in einer Grundwassermeßstelle.

Es werden eine neuartige Durchflußmeßzelle sowie eine Bohrlochsonde beschrieben.

Bei oben genannten Verfahren und Vorrichtungen wird das Meßgas im Kreislauf durch den Diffusionsschlauch und die Meßkammer umgewälzt. Dadurch baut sich entsprechend der Halbwertszeit des Radon-222 von 3,8 Tagen langsam ein Konzentrationsgleichgewicht zwischen dem umgebenden Wasser und dem Meßgas auf. Die relativ lange Halbwertszeit verhindert die Registrierung schneller (im Minutenbereich) Änderungen der Radonkonzentration im Wasser.

V. Labed et al. (Health Physics 63 (2), 172-178, 1992) beschreiben eine Messvorrichtung, in der das Messgas nicht im Kreislauf geführt wird. Allerdings wird darin das Wasser im Kreislauf geführt, da die Vorrichtung nicht der Messung der Radonkonzentration, sondern der Bestimmung der Membranpermeabilität dient.

Der Erfindung liegt die Aufgabe zugrunde, ein Verfahren und eine vorrichtung zur schnellen und kontinuierlichen Erfassung von Änderungen der Konzentration von in Wasser gelöstem Radon-Gas anzugeben, die eine wirtschaftliche, technisch wenig aufwendige Lösung darstellen und in vielen Anwendungsbereichen, sei es mobil oder stationär, die Erfassung schneller Änderungen der Konzentration von Radon im Wasser mit möglichst hoher zeitlicher Auflösung gestatten.

Diese Aufgabe wird erfindungsgemäß gelöst durch die Merkmale im kennzeichnenden Teil der Ansprüche 1 und 7.

Zweckmäßige Ausgestaltungen der Erfindung sind in Unteransprüchen beschrieben.
Ein besonderer Vorteil der Erfindung besteht darin, daß die bei allen bekannten Lösungen vorhandene enorme zeitliche Verzögerung bei der Erfassung der Konzentrationsänderungen des in Wasser gelösten Radon-Gases dadurch verhindert bzw. minimiert wird, daß der Gaskreislauf geöffnet wird.

Im Unterschied zu den bekannten Verfahren und Vorrichtungen, bei denen eine definierte Wassermenge quasistationär einen Diffusionsschlauch umgibt, wird nun erfindungsgemäß ständig radonfreies Meßgas auf der einen Seite des Diffusionsschlauches zugeführt. Das Meßgas nimmt das radioaktive Edelgas Radon, welches aus dem parallel oder im Gegenstrom zum Meßgas auf der anderen Seite des Diffusionsschlauches mit einer optimierten Strömungsgeschwindigkeit ständig neu zugeführten Wasser durch den Diffusionsschlauch hindurch diffundiert, auf und leitet es zu einer geeigneten Meßeinrichtung.

Bei Gewährleistung stabiler Randbedingungen ist die Konzentration von Radon im Meßgas direkt proportional der Konzentration von Radon im Wasser. Bei Verwendung von besonders geeigneten Meßeinrichtungen lassen sich auch bei geringen Aktivitätskonzentrationen von wenigen Bequerel Radon im Liter Wasser zeitliche Auflösungen im Bereich von ca. 2 Minuten und darunter erzielen.

Die Dimensionierung und die geometrische Form der Membran und die für das Wasser und das Trägergas erforderlichen Strömungsgeschwindigkeiten können bei Bedarf entsprechend den konkret vorliegenden Aufgabenstellungen, dem zu überwachenden Konzentrationsbereich und der gewünschten zeitlichen Auflösung optimiert werden.

Dadurch, daß ständig neues, radonfreies Gas, z.B. Luft, durch den vom Wasser umgebenen Gasraum (z.B. Diffusionsschlauch) in das Radonmeßgerät gepumpt, dort kontinuierlich gemessen, und danach an die Umgebung abgegeben wird, wird vermieden, daß sich in dem Meßgas Folgenuklide des Radon über längere Zeit aufbauen können und damit den Meßeffekt zeitlich verzögern.

Erstmals wird hierdurch auch möglich, die Abnahme von Radonkonzentration direkt zu erfassen.

Die Erfindung soll nachstehend anhand von Ausführungsbeispielen näher erläutert werden.

Es zeigen:

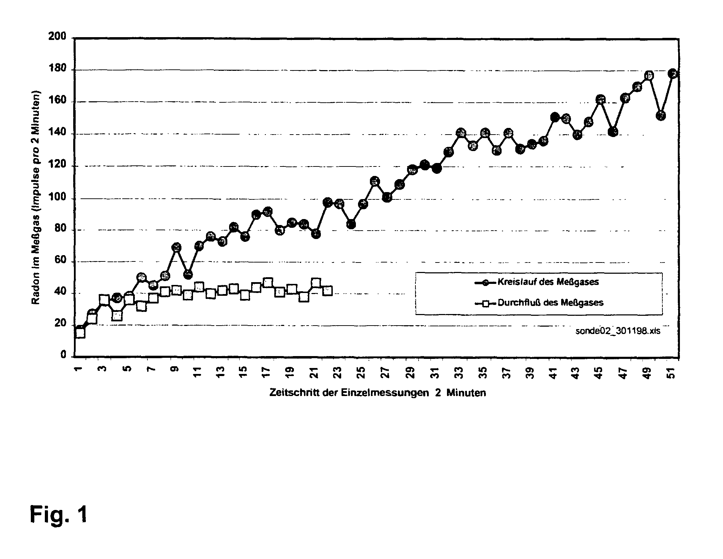

- Fig. 1: Ein Diagramm zum Vergleich zwischen Kreislauf und Durchfluß des Messgases, Durchfluß von ca. 12 Liter Leitungswasser pro Minute durch die Sonde (Spezifische Aktivität: ca. 1 Bq Radon pro Liter Wasser)

Wird, wie in Fig. 1 dargestellt, ein Meßgas im Kreislauf gefördert (Punkte), wie es bei den bisher angewendeten Meßverfahren der Fall gewesen ist, so wird die Meßzeile erheblich kontaminiert und ist nicht mehr in der Lage, geringe Aktivitätsdifferenzen mit der gewünschten Zeitauflösung erfassen zu können. Der Gleichgewichtszustand wird erst nach ca. 2 Stunden erreicht.

Wird ständig neues Meßgas im Durchflußmodus herangeführt (Quadrate), so baut sich nach wenigen Minuten ein konstantes Meßsignal auf, welches der spezifischen Aktivität von Radon im Wasser weitgehend proportional ist, auf kurzfristige Aktivitätsänderungen schnell reagiert und nur geringfügiger Korrekturen bedarf.

Die Erfindung ist nicht beschränkt auf die hier beschriebenen Ausführungsbeispiele. Vielmehr ist es möglich, durch geeignete Kombination der genannten Mittel und Merkmale weitere Ausführungsvarianten zu realisieren, ohne den Rahmen der Erfindung zu verlassen.

## Patentansprüche

1. Verfahren zur kontinuierlichen und schnellen Erfassung von Änderungen der Konzentration von in ständig neu zugeführtem Wasser gelöstem Radongas unter Verwendung wasserdichter, gasdurchlässiger Membranen,
**dadurch gekennzeichnet , dass** ohne Realisierung eines Kreislaufes ständig neues, radonfreies Gas durch einen vom strömenden Wasser umgebenen, durch die wasserdichte, gasdurchlässige Membran abgetrennten Gasraum in ein Radonmessgerät gepumpt und dort kontinuierlich gemessen wird, und dass die Erfassung mit einer zeitlichen Auflösung im Bereich von circa 2 Minuten und darunter erfolgt.

2. Verfahren nach Anspruch 1,
**dadurch gekennzeichnet, dass** das radonfreie Gas Luft ist.

3. Verfahren nach Anspruch 1,
**dadurch gekennzeichnet dass** das Gas nach Verlassen des Radonmessgerätes an die Umgebung abgegeben wird.

4. Verfahren nach Anspruch 1,
**dadurch gekennzeichnet, dass** das Wasser und das Messgas im Gegenstrom entlang der Membran geführt werden.

5. Verfahren nach Anspruch 1,
**dadurch gekennzeichnet, dass** das Wasser und das Messgas parallel zu der Membran geführt werden.

6. Verfahren nach einem der voranstehenden Ansprüche 1 bis 5,
**dadurch gekennzeichnet dass** der Gasraum ein Diffusionsschlauch ist.

7. Vorrichtung zur kontinuierlichen und schnellen Erfassung von Änderungen der Konzentration von in ständig neu zugeführtem wasser gelöstem Radongas,
**dadurch gekennzeichnet, dass** ein vom strömenden Wasser durch eine wasserdichte, gasdurchlässige Membran abgetrennter Gasraum einen Eingang und einen Ausgang aufweist und im strömenden Wasser angeordnet ist, wobei der Eingang des Gasraumes mit einer Gasquelle und der Ausgang des Gasraumes mit dem Eingang eines Radonmessgerätes verbunden ist, und der Ausgang des Radonmessgerätes in die Umgebungsluft mündet, wobei die Vorrichtung weiterhin **dadurch gekennzeichnet ist, dass** sie für die Erfassung mit einer Zeitlichen Auflösung im Bereich von circa 2 Minuten und darunter geeignet ist.

8. Vorrichtung nach Anspruch 7
**dadurch gekennzeichnet, dass** der Gasraum ein Diffusionsschlauch ist.

## Revendications

1. Procédé pour la saisie rapide et continue des variations de concentration de gaz radon dissous dans de l'au continuellement renouvelée, en recourant à des membranes étanches à l'eau et perméables aux gaz,
**caractérisé en ce que** sans réalisation d'un circuit, du gaz renouvelé et exempt de radon est continuellement pompé dans un instrument de mesure du radon par une chambre à gaz entourée d'eau courante, séparée par la membrane étanche à l'eau et perméable aux gaz et y est continûment mesuré, et **en ce que** la saisie est effectuée avec une définition temporelle de l'ordre de 2 minutes ou moins.

2. Procédé selon la revendication 1,
**caractérisé en ce que** le gaz exempt de radon est de l'air.

3. Procédé selon la revendication 1,
**caractérisé en ce que** le gaz est libéré dans l'environnement après avoir quitté l'instrument de mesure du radon.

4. Procédé selon la revendication 1,
**caractérisé en ce que** l'eau et le gaz de mesure s'écoulent à contre-courant le long de la membrane.

5. Procédé selon la revendication 1,
**caractérisé en ce que** l'eau et le gaz de mesure s'écoulent parallèlement à la membrane.

6. Procédé selon l'une des précédentes revendications 1 à 5,
**caractérisé en ce que** la chambre à gaz est un tuyau de diffusion.

7. Dispositif pour la saisie rapide et continue des variations de concentration de gaz radon dissous dans de l'au continuellement renouvelée,
**caractérisé en ce qu'**une une chambre à gaz séparée de l'eau courante par une membrane étanche à l'eau et perméable aux gaz présente une entrée et une sortie et est placée dans l'eau courante, l'entrée de la chambre à gaz étant reliée à une source de gaz, et la sortie de la chambre à gaz à l'entrée d'un instrument de mesure du radon, et la sortie de l'instrument de mesure du radon découche dans l'air ambiant, le dispositif étant en outre caractérisé en ceci qu'il est adapté à la saisie avec une définition temporelle de l'ordre de 2 minutes ou moins.

8. Dispositif selon la revendication 7,
**caractérisé en ce que** la chambre à gaz est un tuyau de diffusion.

## Claims

1. Method for the continual and fast detection of changes of the concentration of radon gas dissolved in constantly and newly supplied water, with the use of water-tight and gas-permeable membranes,
**wherein,**
without the realisation of a cycle, constantly new, radon-free gas is pumped through a gas zone surrounded by water and separated by a water-tight, gas-permeable membrane, into a radon measuring equipment unit where it is continually measured, and that the detection takes place with a time resolution in the range of approximately two minutes or less.

2. Method according to Claim 1,
**wherein**
the radon-free gas is air.

3. Method according to Claim 1,
**wherein**
the gas, after departing from the radon measuring equipment unit, is discharged to the ambient surroundings.

4. Method according to Claim 1,
**wherein**
the water and the measuring gas are conducted in the counter-current along the membrane.

5. Method according to Claim 1,
**wherein**
the water and the measuring gas are conducted parallel to the membrane.

6. Method according to one of the above-mentioned Claims 1 to 5,
**wherein**
the gas zone is a diffusion hose.

7. Device for the continual and, in particular, fast detection of the changes of concentration of radon gas dissolved in water,
**wherein**
a gas zone has an inlet and an outlet and is arranged in flowing water, where the inlet of the gas zone is connected to a gas source and the outlet of the gas zone is connected with the inlet of a radon measuring equipment unit, and the outlet of the radon measuring equipment unit opens out in the ambient air, wherein the device is still **characterised in, that** it is qualified for a detection with a time resolution in the range of approximately two minutes or less.

8. Device according to Claim 7,
**wherein**
the gas zone is a diffusion hose.
